## Europäisches Patentamt
## European Patent Office
## Office européen des brevets

(19)

(11) Veroffentlichungsnummer: **0 136 365**
A1

(12) # EUROPÄISCHE PATENTANMELDUNG

(21) Anmeldenummer: 83109737.3

(22) Anmeldetag: 29.09.83

(51) Int. Cl.⁴: **A 61 B 1/00**
**A 61 B 1/06, G 02 B 6/06**

(43) Veröffentlichungstag der Anmeldung:
10.04.85 Patentblatt 85/15

(84) Benannte Vertragsstaaten:
AT BE CH DE FR GB IT LI NL SE

(71) Anmelder: Storz-Endoskop GmbH
Kreuzgutweg 22
CH-8207 Schaffhausen(CH)

(72) Erfinder: Storz, Karl
Auf dem Schildrain 39
D-7200 Tuttlingen(DE)

(74) Vertreter: Wenzel, Joachim, Dipl.-Ing.
Hauptmannsreute 46
D-7000 Stuttgart 1(DE)

(54) **Endoskopisches Sehrohr.**

(57) Die Erfindung betrifft ein endoskopisches Sehrohr, das starr ausgebildet und mit einem Licht-Übertragungskabel aus Glasfasern versehen ist.

Um die Stabilität eines solchen Sehrohres wesentlich zu erhöhen, ist erfindungsgemäß zwischen den einzelnen Glasfasern (1) des Kabels ein Kitt (2) angeordnet. Auf diese Weise entsteht ein nahezu starrer Außenmantel für das Hüllrohr des endoskopischen Sehrohres. Die Stabilität auf Biegung ist wesentlich erhöht und das Sehrohr mit einem sehr kleinen Durchmesser insgesamt wesentlich härter.

Derartige Sehrohre finden vor allem in der Medizintechnik Anwendung.

FIG.3

EP 0 136 365 A1

Storz-Endoskop GmbH, Schaffhausen

Endoskopisches Sehrohr

Die Erfindung bezieht sich auf ein endoskopisches Sehrohr nach dem Oberbegriff des Anspruchs 1.

Derartige endoskopische Sehrohre finden vor allem in der Medizintecknik Anwendung und müssen in den meisten Fällen einen möglichst kleinen Durchmesser haben, weil die Sehrohre noch in einen Endoskopschaft eingeschoben werden müssen, der ebenfalls einen möglichst kleinen Durchmesser haben soll. Die bekannten endoskopischen Sehrohre zeigen zum Beispiel einen Außendurchmesser von nur 2 mm. Dabei ist innerhalb dieser Außenhülle mit einem so kleinen Durchmesser bekanntermaßen nicht nur das Linsensystem angeordnet, das der Beobachtung dient, sondern zusätzlich muß auch noch das erwähnte Lichtleitkabel parallel hierzu bis zum Objektiv geführt werden, um das Objekt anzustrahlen. Dabei ummantelt das Glasfaserbündel zum Beispiel das in der Mitte liegende Stablinsensystem, welches von einem dünnwandigen Hüllrohr umgeben ist, das zum Beispiel einen Durchmesser von nur 1 mm haben kann.

-/-

Darüberhinaus ist auch bekannt, das Bild selbst durch ein Glasfaserbündel zu leiten.

Infolge des erwähnten kleinen Durchmessers sind die bekannten starren endoskopischen Sehrohre sehr empfindlich. Allein durch eine unachtsame Handhabung im Krankenhaus kann zum Beispiel die erwähnte Stablinse brechen, weil die Biegefestigkeit nur sehr gering ist. Die erwähnte Außenhülle des endoskopischen Sehrohres besteht bekanntermaßen aus einem dünnwadigen Metallrohr, das natürlich biegsam ist. Bei einer solchen Biegung bricht aber dann vielfach zumindest eine der erwähnten Stablinsen mit dem sehr kleinen Durchmesser.

Der Erfindung liegt die Aufgabe zugrunde, diesen schweren Nachteil zu beheben und das endoskopische Sehrohr der eingangs erwähnten Art so zu verbessern, daß die Stabilität des Sehrohres insgesamt wesentlich erhöht ist.

Zur Lösung dieser Aufgabe sind die kennzeichnenden Merkmale des Anspruchs 1 vorgesehen. Dadurch werden die einzelnen Glasfasern mit einer Stärke von zum Beispiel 30 $\mu$m miteinander verkittet, so daß sie praktisch einen nahezu starren Außenmantel für das Hüllrohr des endoskopischen Sehrohres

-/-

bilden und dieses somit schützen. Auf diese Weise wird
die Stabilität auf Biegung wesentlich erhöht und das
Sehrohr mit dem sehr kleinen Durchmesser insgesamt wesentlich härter.

Zur Fertigung sind die kennzeichnenden Merkmale des Anspruchs 2 zweckmäßig. Dadurch kann erreicht werden, daß
der Kitt auch in die kleinsten Hohlräume zwischen den
einzelnen Glasfasern eindringt, zumal der Kitt am distalen
Ende des Sehrohres teilweise wieder austritt, wobei natürlich auch die eingeschlossene Luft herausgedrückt
wird.

Weitere Vorteile und Einzelheiten der Erfindung ergeben
sich aus der nun folgenden Beschreibung eines Ausführungsbeispiels unter Hinweis auf die Zeichnung. In dieser zeigen:

-/-

- 4 -

Fig. 1 eine Seitenansicht auf das endoskopische Sehrohr insgesamt;

Fig. 2 einen Schnitt nach der Linie AB durch das Sehrohr nach Fig. 1 in stark vergrößerndem Maßstab und

Fig. 3 einen Ausschnitt aus der Faseranordnung nach Fig. 2 in dem gegenüber vergrößerndem Maßstab.

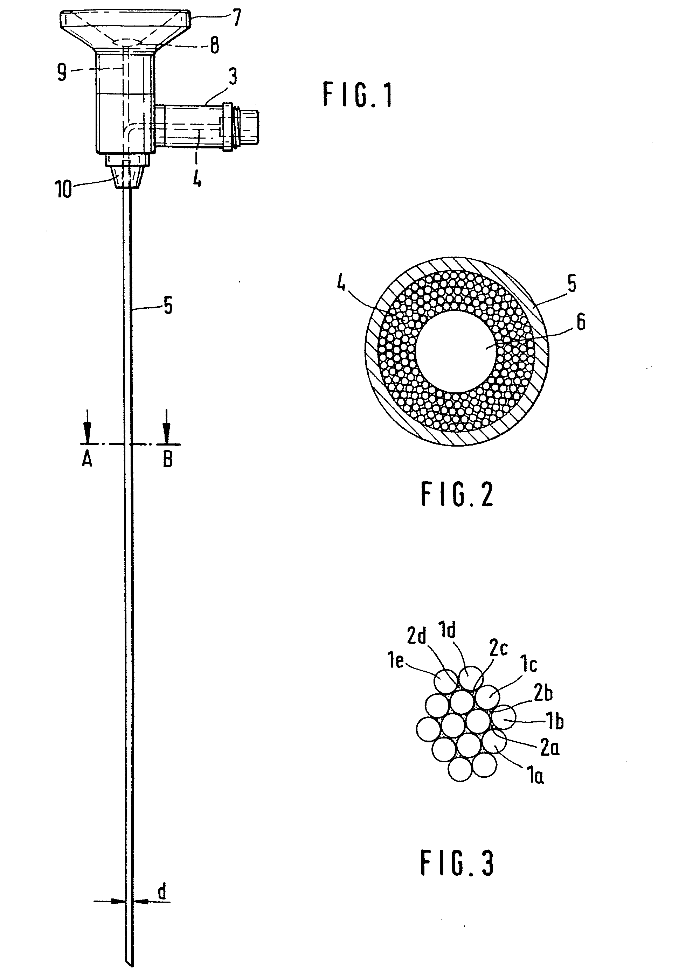

Die Seitenansicht von außen gemäß Fig. 1 unterscheidet sich nicht von der Seitenansicht auf ein herkömmliches endoskopisches Sehrohr. Oben sieht man das Okular 7, in der mit unterbrochenen Linien die Okular-Linse 8 angedeutet ist. Von dieser führt die ebenfalls mit unterbrochenen Linien eingezeichnete Bohrung 9 senkrecht nach unten. Rechts sieht man einen Anschluß 3 in bekannter Weise für das Lichtkabel 4, welches ebenfalls mit unterbrochenen Linien angedeutet ist. Dies wird nun in die erwähnte Bohrung 9 hineingeführt, wo es das Hüllrohr des optischen Systems 6 ummantelt, wie in Fig. 2 stark vergrößert dargestellt ist.

Weiter unten ist in dem Konus 10 die Außenhülle 5 des Sehrohres befestigt, die einen Durchmesser d von z. B. nur 2 mm hat.

-/-

- 5 -

Fig. 2 zeigt den Schnitt nach der Linie A-B sehr stark vergrößert. Die Außenhülle 5 des endoskopischen Sehrohres kann aus dünnwandigem Metallrohr in bekannter Weise bestehen.

Das Glasfaserbündel 4 zeigt nur schematisch eine Reihe von Glasfasern, die z. B. einen Durchmesser von 30 µm haben können. Wie ersichtlich, ummanteln die Glasfasern das Hüllrohr 6, das etwa in der Mitte des Glasfaserbündels angeordnet ist. Nicht auszuschließen ist aber auch, daß das optische System 6 ein wenig oder sogar stark exzentrisch gegenüber dem Glasfaserbündel 4 angeordnet ist.

Schließlich zeigt Fig. 3 einige wenige Glasfasern im Querschnitt demgegenüber nochmals stark vergrößert. In den Zwischenräumen zwischen diesen Glasfasern 1 a bis 1 e ist nun erfindungsgemäß ein bekannter optischer Kitt 2 a bis 2 d angeordnet. Dies ist hier nur beispielhaft und schemahaft gezeigt. In Wahrheit ist der Kitt in sämtlichen Zwischenräumen zwischen den einzelnen Glasfaserquerschnitten vorhanden und auch außerherum. Dies bedeu-

-/-

tet, daß der Kitt sowohl zwischen dem Hüllrohr 6 und dem Glasfaserbündel 4 als auch zwischen dem Außenmantel 5 und dem Glasfaserbündel 4 vorhanden ist und dadurch dem Glasfaserbündel 4 eine sehr große Festigkeit gibt, die zuvor gar nicht vorhanden war, weil gemß dem Stand der Technik die einzelnen Glasfasern nur lose im Bündel vorliegen und somit überhaupt gar keinen Beitrag zur Festigkeit des endoskopischen Sehrohres in diesem Bereich beitrugen.

Durch den Kitt stellt aber nunmehr das Glasfaserbündel 4 eine kompakte starre Masse dar, die das Hüllrohr 6 auch dann z. B. vor einem Biegebruch schützt, wenn diese exzentrisch gegenüber dem Glasfaserbündel 4, wie schon erwähnt, angeordnet ist.

Die Fertigung des Erfindungsgegenstandes ist verhältnismäßig einfach. Es ist lediglich erforderlich, in den Anschluß 1 den bekannten optischen Kitt in flüssigem Zustand unter Druck einzuführen. Nachdem er sämtliche Hohlräume zwischen dem Anschluß 3 und dem distalen Ende des Sehrohres ausgefüllt hat, tritt der Kitt dann am distalen Ende aus. Dabei hat er die eingeschlossene Luft vor sich hergeschoben. Auf diese Weise ist möglich, die Stabilität

- 7 -

eines jeden bekannten endoskopischen Sehrohres wesentlich zu erhöhen. Bekanntlich härtet der Kitt nach einer gewissen Zeit aus und bildet dann zusammen mit dem Glasfaserbündel 4 eine starre Masse, die sich nicht leicht biegen läßt, so daß die Optik 6 wesentlich besser als bisher geschützt ist.

- 8 -

## Ansprüche

1. Endoskopisches Sehrohr, das starr ausgebildet und mit einem Licht-Übertragungskabel aus Glasfasern versehen ist, dadurch gekennzeichnet, daß zwischen den einzelnen Glasfasern (1a - 1e) des Kabels (4) ein Kitt (2a - 2d) angeordnet ist.

2. Endoskopisches Sehrohr nach Anspruch 1, dadurch gekennzeichnet, daß der Kitt (2a - 2d) in flüssigem Zustand unter Druck in den Anschluß (3) für das Lichtleitkabel (4) eingeführt wird.

0136365

1/1

FIG.1

FIG.2

FIG.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 3) |
|---|---|---|---|
| X | FR-A-2 397 930 (AMERICAN HOSPITAL SUPPLY CORP.) * Insgesamt * | 1,2 | A 61 B 1/00 A 61 B 1/06 G 02 B 5/17 |
| X | FR-A-2 390 748 (AMERICAN HOSPITAL SUPPLY CORP.) * Spalte 5, Zeile 32 - Spalte 6, Zeile 22; Figuren 1-3 * | 1 | |
| X | US-A-3 669 772 (R.R. STRACK) * Spalte 2, Zeile 67 - Spalte 3, Zeile 10; Figuren 1,2 * | 1 | |
| X | FR-A-1 560 249 (F. FORT) * Insgesamt * | 1 | |
| A | CH-B- 389 938 (AMERICAN OPTICAL COMP.) * Seite 2, Zeile 34 - Seite 4, Zeile 24; Figuren 1-7 * | 1 | RECHERCHIERTE SACHGEBIETE (Int. Cl. 3) A 61 B G 02 B |
| A | US-A-3 799 150 (L. BONNET) * Spalte 2, Zeilen 1-30; Figuren 1-3 * | 1 | |
| A | US-A-3 498 286 (M.L. POLANYI) * Insgesamt * | 1 | |

-/-

Der vorliegende Recherchenbericht wurde für alle Patentansprüche erstellt.

| Recherchenort DEN HAAG | Abschlußdatum der Recherche 18-05-1984 | Prüfer ZILLIOX J.M. |
|---|---|---|

**Europäisches Patentamt**

**EUROPÄISCHER RECHERCHENBERICHT**

**0136365**

Nummer der Anmeldung

EP 83 10 9737

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 461 332 (OLYMPUS OPTICAL CO.) <br> * Seite 5, Zeile 14 - Seite 6, Zeile 22; Figuren 1-6 * <br> --- | 1,2 | |
| A | US-A-3 581 376 (H.N. PILLING) <br> * Spalte 3, Zeile 5 - Spalte 4, Zeile 21; Figuren 1-5 * <br> ----- | 1 | |
| | | | RECHERCHIERTE SACHGEBIETE (Int. Cl. 3) |

Der vorliegende Recherchenbericht wurde für alle Patentansprüche erstellt.

| Recherchenort | Abschlußdatum der Recherche | Prüfer |
|---|---|---|
| DEN HAAG | 18-05-1984 | ZILLIOX J.M. |